(19) Europäisches Patentamt
European Patent Office
Office européen des brevets

(11) EP 1 166 784 B1

(12) **EUROPEAN PATENT SPECIFICATION**

(45) Date of publication and mention of the grant of the patent:
**20.02.2008 Bulletin 2008/08**

(21) Application number: **00913083.2**

(22) Date of filing: **03.04.2000**

(51) Int Cl.:
***A61K 31/409*** *(2006.01)* ***A61K 41/00*** *(2006.01)*
***A61P 9/00*** *(2006.01)*

(86) International application number:
**PCT/JP2000/002156**

(87) International publication number:
**WO 2000/059505 (12.10.2000 Gazette 2000/41)**

(54) **INHIBITION OF VASCULAR RESTENOSIS AFTER ANGIOPLASTY**

INHIBITION DER VASKULÄREN RESTENOSE NACH EINER ANGIOPLASTIE

INHIBITION DE LA RESTENOSE VASCULAIRE APRES UNE ANGIOPLASTIE

(84) Designated Contracting States:
**AT BE CH CY DE DK ES FI FR GB GR IE IT LI LU MC NL PT SE**

(30) Priority: **02.04.1999 JP 9565499**

(43) Date of publication of application:
**02.01.2002 Bulletin 2002/01**

(73) Proprietor: **Meiji Seika Kaisha, Ltd.**
**Tokyo 104-8002 (JP)**

(72) Inventors:
- **NAGAE, Tsuneyuki**
  **Tokyo Medical College**
  **Tokyo 160-0022 (JP)**
- **AIZAWA, Katsuo**
  **Tokyo Medical College**
  **Tokyo 160-0022 (JP)**

(74) Representative: **Le Coupanec, Pascale A.M.P. et al**
**Nony & Associés,**
**3 rue de Penthièvre**
**75008 Paris (FR)**

(56) References cited:
**WO-A-00/02882** **WO-A1-95/03797**
**US-A- 5 308 861**

- **SAITO, T.; HAYASHI, J.; AIZAWA, K.: "Acute Effects of Photodynamic Treatment on Elastic Fibre Network in Atherosclerotic Plaques of Rabbit Aorta" LASERS IN MEDICAL SCIENCE, vol. 13, 1998, pages 126-131, XP001070452 London**
- **STERNBERG E D ET AL: "Porphyrin-based Photosensitizers for Use in Photodynamic Therapy" TETRAHEDRON, ELSEVIER SCIENCE PUBLISHERS, AMSTERDAM, NL, vol. 54, no. 17, 23 April 1998 (1998-04-23), pages 4151-4202, XP004113291 ISSN: 0040-4020**
- **LEVY J G: "Photodynamic therapy" TRENDS IN BIOTECHNOLOGY, ELSEVIER, AMSTERDAM, NL, vol. 13, no. 1, January 1995 (1995-01), pages 14-18, XP004207115 ISSN: 0167-7799**
- **WILLIAM D. COATS JR. ET AL: 'Tin ethyl etiopurpurin signifinanctly inhibits vascular smooth muscle cell proliferation in vivo' BIOCHEMISTRY AND CELL BIOLOGY vol. 74, no. 3, 1996, pages 325 - 331, XP002927497**
- **P.C. DARTSCH, ET AL: 'Photodynamic therapy of vascular stenoses? Response of cultured human smooth muscle cells from non-atherosclerotic arteries and atheromatous plaques following treatment with photosensitizing porphyrins' PROCEEDINGS. SPIE-THE INTERNATIONAL SOCIETY FOR OPTICAL ENGINEERING vol. 1462, 1990, pages 77 - 80, XP002927498**
- **PETER C. DARTSCH ET AL: 'Responses of cultured smooth muscle cells from human nonatherosclerotic arteries and primary stenosing lesions after photoradiation: implications for photodynamic therapy of vascular stenoses' JOURNAL OF THE AMERICAN COLLEGE CARDIOLOGY vol. 15, 1990, pages 1545 - 1550, XP002927499**
- **KAZUO UMEMURA: 'PTCA go no sai sakushu: Yakubutsu chiryou no kanousei' KEKKAN TO NAIHI vol. 6, no. 1, 1996, pages 56 - 64, XP002935469**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

- **JENKINS M.P. ET AL: 'INTRA-ARTERIAL PHOTODYNAMIC THERAPY USING 5-ALA IN A SWINE MODEL' EUROPEAN JOURNAL OF VASCULAR AND ENDOVASCULAR SURGERY vol. 4, no. 16, 1998, pages 284 - 291**
- **62 Annual Scientific Meeting of Japanese Circulation Society. Abstracts, p. 465 (1998)**


Remarks:

The file contains technical information submitted after the application was filed and not included in this specification

## Description

This invention relates to a use of a photosensitive agent for the preparation of a vascular restenosis- suppressing composition which exhibits a therapeutic effect of suppressing a thickening of the vascular intima of the blood vessel at a site thereof having received an angioplasty treatment, in a photochemotherapy or photodynamic therapy (abbreviated to PDT) to be performed for the purpose of suppressing the vascular restenosis of the angioplasty-dilated site of the blood vessel which is inducible due to a thickening of the vascular intima of the blood vessel, after the narrowed or stenosed site of the blood vessel had been dilated by an angioplasty treatment according to percutaneous transluminal coronary angioplasty (abbreviated as PTCA) or percutaneous transluminal angioplasty (abbreviated as PTA).

BACKGROUND ART

Photodynamic therapy (hereinafter, referred to as PDT) is a chemotherapeutic method which comprises initially administering such a photosensitive substance that is activatable by irradiation with ultraviolet ray or laser light; and then activating the administered photosensitive substance, for example, by irradiation of the light or laser to said photosensitive substance present in the lesion of the living body where the administered photosensitive substance has accumulated, or said photosensitive substance present in the blood containing the photosensitive substance, so as to produce the cytotoxic effect of the photosensitive substance, whereby said lesion is treated phototherapeutically (Ann. Vasc. Surg., Vol. 9, pp.80-86 (1999) and Cardicovasc. Surg., Vol. 3, pp.489-494 (1995).

Japanese Patent Publication No. Hei-6-88902 and No. Hei-6-89000 describe the use of fluorescent tetrapyrrole derivatives, for example, mono-L-aspartylchlorin e6 and mono-L-glutamylchlorin e6 or salts thereof, as a photosensitive agent for the diagnosis and therapeutic treatment of tumor by PDT.

Mono-L-aspartylchlorin e6 is the compound represented by the formula (Ia)

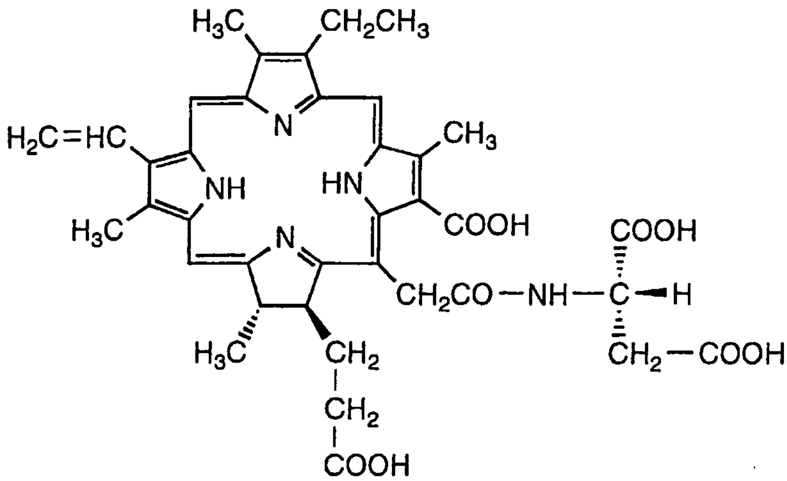

(Ia)

Mono-L-glutamylchlorin e6 is the compound represented by the formula (Ib)

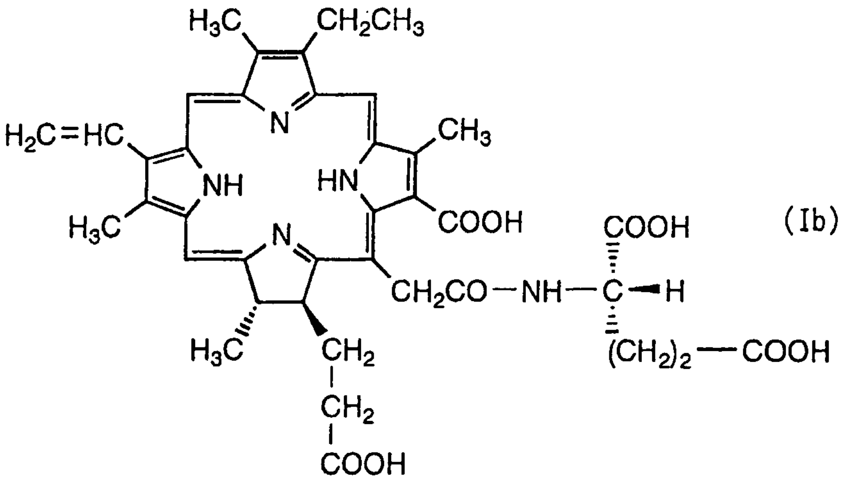

(Ib)

The above-described fluorescent tetrapyrrole compounds have been reported to be used as the photosensitive agent in PDT for arteriosclerosis, because they can effect their selective accumulation in the lesions of arteriosclerosis, including such coronary arteriosclerosis which can induce myocardial infarction and angina, as well as such cerebral arteriosclerosis which can induce transient cerebral ischemia and cerebral infarction. Said tetrapyrrole compounds have been reported to exhibit some therapeutic effects for the arteriosclerosis (Japanese Patent Laid-Open publication No. Hei-4-330013 and U.S. Patent No. 5308861).

On the other hand, in the treatment of ischemic arterial diseases induced by the arteriosclerosis, a treatment by PTCA or PTA is generally used for dilating the narrowed or stenosed sites or lesions of arterial vessels. For this purpose, an intravascular catheter may be used. There is, however, a problem that after the angioplasty operations, a vascular restenosis can be induced at the angioplasty-dilated site of the blood vessel at a rate of from 30% to 60% of the angioplasty operations. Vascular restenosis occurs due to a neo-intimal thickening which is caused by proliferation and migration of the smooth muscle cells in the tunica media of blood vessel at said site. The mechanism of onset of the neo-intimal thickening is now postulated to be as follows. Thus, during the process of dilating the narrowed or stenosed site of the vascular vessel having the arteriosclerotic lesions which was effected by means of a balloon catheter according to PTCA or PTA, it occurs that the vascular endothelial cells and their surrounding tissues at said site are intervened and injured by the inflated balloon of the balloon catheter and thus the subendothelial tissues of the blood vessel wall at said site are barely exposed, resulting in the activation of platelets. As an outcome of the biological reactions of repairing the balloon-injured site at said lesions, the so activated platelets can adhere to the balloon-injured site and can agglutinate, and will release the smooth muscle growth factors, such as PDGF (platelet-derived growth factor) and TGF-β (transforming growth factor). In addition, macrophages can infiltrate into said balloon-injured site and release a variety of the growth factors. These biological processes can trigger the differentiation, proliferation, and migration of the smooth muscle cells in the tunica media at said site of the blood vessels, which can further involve stimulations of the intimal smooth cells for forming the vascular neo-intima, so that the intimal smooth cells themselves will release the growth factors. In this way, an intimal thickening which lasts for a long period of time can be produced in the blood vessel at said site. As a result, the vascular restenosis can be involved (a Japanese journal: Kekkan to Naihi, Vol.6, pp.56-64 (1996).

Up to now, it has been found from certain animal tests that some of drugs, such as antiallergic drugs, ACE-inhibitors, angiotensin II-antagonists, antiplatelet drugs, PDE III-inhibitors, have a suppressive effect on the vascular intimal thickening which is induced post to the PTCA or PTA treatment (Japanese Patent Laid-Open publications No. Hei-9-188619, No. Hei-8-176013 and No. Hei-7-25768).

The mechanism of the pharmacological action of the above-mentioned drugs as tested comprises suppression and inhibition against the processes of the mechanism of onset of the vascular intimal thickening which are described in the above. However, the mechanism of the pharmacological action of the tested drugs above can be developed in different ways between animals and human, resulting in some great difference in the medicinal efficacy of the above drugs. Currently, in the therapeutic process for preventing the vascular restenosis which is conducted in clinical practice, there is carried out also a variety of supplementary therapies by administration of drug and occasionally concomitant insertion of a stenting device is done, in order to ensure that a favorable outcome can be attained. Still, however, the thus attainable outcome is not yet satisfactory. Therefore, there remains a demand for providing a more effective therapeutic method which is able to suppress surely the vascular restenosis at a site of the blood vessel having received a PTCA or PTA treatment (Dtsch. Med. Wschr, Vol. 123, pp.840-846 (1998).

On the other hand, as the photosensitive agent for PDT, Photofrin is currently used in the clinical treatment of cancer. When Photofrin is used, it is necessary that a time interval to be interposed between the administration of Photofrin and the commencement of light irradiation should be adjusted to be of a time period of 48 to 72 hours. Photosensitive reactions which would be caused in the patients by the administered Photofrin can continue longly for four weeks, and therefore it is required that the time period necessary for the shielding of the patients from lights should be kept for 4 weeks after the administration of Photofrin. This can result in that the hospitalization of patients must be made for a considerably long time period and can lower QOL (Quality of Life) of the patients (THE JOURNAL OF JAPAN SOCIETY FOR LASER SURGERY AND MEDICINE, Vol. 18, pp.295-300 (1997). While, when mono-L-aspartylchlorin e6 or its tetrasodium salt is used for PDT for the cancer treatment, the time interval to be interposed between the drug administration and the commencement of laser irradiation can advantageously be adjusted to be as short as 4 to 6 hours, and further the time period as required to attain the disappearance of the photosensitive reactions caused by the administered chlorin e6 compound may be shorter in the order of two weeks, which is a half of that required for Photofrin.

The present inventors had previously reported such a therapeutic method for suppressing the vascular restenosis by application of PDT, which method comprised effecting an intravascular irradiation of laser light onto the blood vessel at a site thereof having received an angioplasty treatment (for example, according to PTCA or PTA), with using such a cylindrical optical fiber for the intravascular laser irradiation that had been inserted into and kept in the blood vessel (see The 62nd Scientific Meeting of Japanese Circulation Society, Abstract, p.465 (1988)). The above-mentioned therapeutic method as previously reported by the present inventors, however, has not yet attained such therapeutic effect that a complete and steady suppression can successfully be achieved against the vascular restenosis of the blood vessel as dilated by a PTCA or PTA treatment, which is inducible at the intravascular site having received the PTCA or PTA treatment.

Therefore, there remains a demand for providing a new use of a photosensitive agent for the preparation of a vascular restenosis-suppressing pharmaceutical composition which is suitable in a photodynamic therapy method for suppressing the vascular restenosis inducible after angioplasty such as PTCA or PTA, and which method is able to treat selectively in a simple and efficient way solely the blood vessel site having received the angioplasty treatment, according to a PDT process comprising intravascular irradiation of laser, and which method also can readily be applied in clinics.

## DISCLOSURE OF THE INVENTION

Thus, the present inventors have now made investigations to provide such new use of a suppressant or preventive agent for the preparation of a composition for treating the vascular restenosis, which is able to meet the above-mentioned demands. As a result, the present inventors have now found that, when the above-mentioned mono-L-aspartylchlorin e6 or its tetrasodium salt is intravenously administered to a patient whose blood vessel has received an angioplasty treatment by PTCA or PTA, and when the dose of administration of said chlorin e6 compound for adult patient is adjusted to a dosage of 0.01-50 mg/kg, preferably a dosage of 0.1-5 mg/kg, the so administered mono-L-aspartylchlorin e6 or its tetrasodium salt is able to accumulate preferentially in the smooth muscle cells in the tunica media and intima of the blood vessel at a site thereof having received the PTCA or PTA treatment within a time of 10 minutes to 6 hours after the administration of said chlorin e6 compound. The present inventors now have also found that the amount (or concentration) of the mono-L- aspartylchlorin e6 which has thus accumulated in the layers of the vascular smooth muscle cells at said PTCA or PTA-treated site is sufficient, upon having received the intravascular irradiation of laser at 664 nm, to generate and exert stably such therapeutic effects that the intimal thickening of the blood vessel is inhibited at the intravascular site having received the PTCA or PTA treatment, with suppressing the vascular restenosis inducible at said site.

Thus, in a first aspect of the invention, there is provided a use of a photosensitive agent for the preparation of a vascular restenosis-suppressing composition, intended to be used in combination with a laser irradiating device comprising a balloon catheter to be positioned firmly on an inner wall of a blood vessel at an angioplasty-dilated site of a blood vessel having received a percutaneous transluminal coronary angioplasty or a percutaneous transluminal angioplasty, by means of a photodynamic therapy process comprising intravascular irradiation of laser, and which composition exhibits a suppressing effect on a thickening of the vascular intima in the interior of the blood vessel wall at the angioplasty-dilated site of the blood vessel, characterized in that said composition comprises mono-L-aspartylchlorin e6 of formula (I)

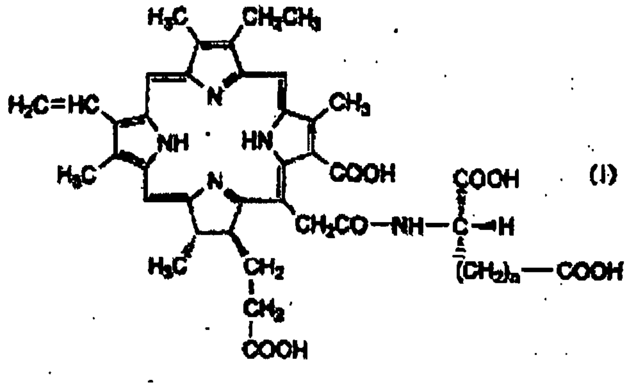

(I)

wherein n is an integer of 1, or a pharmacologically acceptable salt thereof, as an active ingredient.

Of the compounds having the above general formula (I), the compound where n=1 is such one in which L-aspartic acid is coupled at its amino group, with a group -CH2COOH present as a side chain of the tetrapyrrole ring, via the amido-linkage, and which compound is mono-L-aspartylchlorin e6. Preferably, this compound is in the form of tetrasodium salt at the four carboxyl groups thereof.

The compound of general formula (I) which is used as an active ingredient in the composition of this invention may combine with a base to form a salt. Its salts which may be formed by reaction of the chlorin e6 compound of the formula (I) with a base include those with sodium, potassium, calcium, magnesium, ammonium, triethyl-ammonium, trimethylammonium, morpholine, and piperidine.

Disorders for which the composition of this invention is useful may be such vascular restenosises which are inducible at any dilated sites of the narrowed or stenosed parts of the blood vessels in various tumors or in the disordered ocular fundus with age-related macular degeneration, as well as the stenosed parts of the blood vessel with atherosclerosis in the peripheral blood vessels of limbs, coronary artery and cerebral artery. Such vascular restenosis may include, for example, the vascular restenosis inducible after surgical operations of implanting a stenting device, venous graft or artificial blood vessel, as well as the vascular restenosis as resulted from the vascular intimal thickening caused by the treatment according to angioplasty.

The compound of formula (I) usable as an active ingredient in the composition of this invention can be administered orally or parenterally by intravenous or intramuscular injection. It can be also administered percutaneously. For example, the composition of this invention may contain the compound of the active ingredient in the form of its sodium salt and may be formulated as a lyophilized and sterile preparation which is free from pyrogen.

With the composition of this invention for oral administration, the active ingredient compound may be mixed with a solid or liquid carrier and may be formulated in the form of tablets, buccal tablets, troches, capsules, suspensions, or syrups.

A proportion of the chlorin e6 compound as the active ingredient contained in the composition of this invention may depend on a dosage form, but may conveniently be in the range of about 2-60% by weight of a unit dosage.

Preferred forms of the injectable preparation made of the composition of this invention include a sterile solution or a dispersion containing the chlorin e6 compound as the active ingredient, as well as a sterile, lyophilized preparation. Preferably, the carrier may be, for example, water, ethanol, glycerol, propylene glycol, or vegetable oil. In many cases, preferably, an isotonic agent such as sugar or sodium chloride, may be incorporated.

In the case of the injectable preparation made of the composition of the invention, there may further be added such an agent for delaying the absorption of the chlorin e6 compound as the active ingredient, which is, for example, aluminum monostearate or gelatin.

The dosage of the administration of the chlorin e6 compound, which is used as the active ingredient in the composition of the invention, may depend upon the purpose of the therapeutic treatment, but the active chlorin e6 compound may be administered usually at a dosage of 0.01-50 mg/kg of body weight, preferably at a dosage of 0.1-5 mg/kg of body weight at one time for adults.

The PDT method may be conducted in such way that the intravascular irradiation of light is applied to the angioplasty-dilated site of the originally stenosed lesion of the blood vessel at the end of a time lapse of several minutes to tens of hours, preferably 0.5-6 hours after the administration of mono-L-aspartylchlorin e6 or its tetrasodium salt was made. The light-irradiating source for PDT is not limited specifically, but is preferably a laser ray-irradiating source.

For the irradiation of the laser ray, a strong and continuous laser-generating source with a filter, or an activated pigment or other laser-source, in association with a laser-delivery system is used. As the irradiating laser, a laser is used at the wavelength of 360-760 nm, preferably 560-760 nm, more preferably 664 nm. The irradiance of the laser may suitably be selected, and is usually in the range of 10-1000 mW/$cm^2$, preferably 20-500 mW/$cm^2$. The fluence (J/$cm^2$)

of the laser may be calculated to express a mathematical product of the irradiance (W/cm$^2$) by the irradiation time (sec.) of the irradiating laser, in term of the mathematical product that is J = W x sec. In the case when the tumor treatment was made by PDT, a fluence of 50-200 J/ cm$^2$ was usually used for the laser. In contrast, a lesser fluence of 1-100 J/ cm$^2$ is enough for the laser irradiation which is intended to suppress the vascular restenosis by means of the composition according to this invention.

As stated above, the therapeutically effective and sufficient amount of mono-L-aspartylchlorin e6 or its tetrasodium salt as administered is able to accumulate preferentially just in the blood vessel wall at the PTCA or PTA-treated site thereof, already at such a time point at which a relevant time period has lapsed after the administration of said chlorin e6 compound given at its appropriate dose to the patient having the blood vessel treated by PTCA or PTA.

Mono-L-aspartylchlorin e6 or its tetrasodium salt, which has thus accumulated in the wall of the blood vessel at the site having received the PTCA or PTA treatment, is able to be photo-activated with the irradiating laser, when the chlorin e6 compound has received a sufficient fluence of the laser by the intravascular irradiation of the laser which has passed through an optical fiber provided in such a laser-irradiating device that had been constructed in the form of an intravascular catheter and that has been inserted and retained in the blood vessel having received the PTCA or PTA treatment; and also when the chlorin e6 compound as administered has continued to be remaining in the cells of the blood vessel wall at the site having received the PTCA or PTA treatment.

The chlorin e6 compound so photo-activated with laser which is remaining in the cells of said blood vessel wall at the PTCA or PTA-treated site is then able to generate and exert the aforesaid cytotoxic effect on the smooth muscle cells in the tunica media and intima of the blood vessel walls at said site, and is thus able to bring about the suppressing effect against the intravascular intimal thickening and hence the suppressive effect against the vascular restenosis.

However, there occurs such problem that the chlorin e6 compound which has accumulated in the cell layers of the blood vessel wall would not be able to generate their intended effects, so long as the chlorin e6 compound present in said cell layers should have not yet received the irradiating laser at a high laser fluence which would be necessary and sufficient to bring about the photo-activation of said chlorin e6 compound. In addition, it has now been found that, as long as the aforesaid cylindrical optical fiber employed in the previous method is used for the laser irradiation as reported previously, necessary centering of the aforesaid cylindrical optical fiber within the blood vessel, which is to be done for placing the longitudinal axis of the cylindrical optical fiber to be positioned just coincidently with the central axis of the blood vessel lumen, is difficult to be achieved. It has also be observed that if said centering of the cylindrical optical fiber within the blood vessel has not been achieved, then the transmission of the irradiating laser could also be interfered by the bloodstream (and the drug present in the blood) so as to disturb the arrival of the irradiating laser to the blood vessel inner wall; and that consequently, the suppressive effects of the photoactive chlorin e6 compound on the vascular restenosis, which will be obtainable by the use of the aforesaid cylindrical optical fiber, can be varying greatly and become to be too insufficient, depending upon where the particular location of the blood vessel site is to be treated.

Accordingly, there are incurred further problems which need to be resolved and which require the present inventors to try to discover such conditions for PDT that can ensure the laser to be irradiated at a constant and steady fluence uniformly within the blood vessel during the process of the PDT. These problems necessitate the present inventors to develop such a new method which can ensure the vascular restenosis-suppressing effects of mono-L-aspartylchlorin e6 to be achieved and exerted to the full extent.

To solve the above problems, the present inventors have now conducted further investigation. As a result of the extensive investigation, it has now been found and confirmed that, when an effective amount of mono-L-aspartylchlorin e6 is administered as the photoactive agent for PDT; and when there are subsequently conducted such procedures of PDT, wherein use is made of a laser-irradiating device which comprises a known type of balloon catheter having therein a central and longitudinal hole and which device is equipped with a laser-irradiating optical fiber of a thin diameter as provided in the balloon catheter and so arranged as to extend within and through said central hole of the balloon catheter; wherein said laser-irradiating device is inserted in the blood vessel and be held in such a position that the balloon of the balloon catheter of said device as inserted in the blood vessel is placed to be located oppositely to the PTCA or PTA-treated site of the blood vessel; wherein the balloon of the catheter is then inflated by delivery of an inflating liquid medium thereinto so that the inflated balloon so produced of the balloon catheter can have generated the force for supporting the balloon catheter firmly on the blood vessel inner wall, thereby to allow the longitudinal axis of the optical fiber provided in the balloon catheter of said device to be held coincidently with and in the same position as the longitudinal axis of the vascular lumen of said blood vessel at the angioplasty-treated site thereof; wherein there are thus produced such resultant conditions that the inflated balloon of the balloon catheter can have removed the unfavorable interference by the blood stream which is to disturb a uniform, intravascular irradiation of the laser emitting from the optical fiber provided in the balloon catheter; and wherein the intravascular irradiation of the laser for PDT is next effected under the said resultant conditions, it is only then made feasible that the administered chlorin e6 compound can be photo-activated sufficiently to stably and evenly bring about and exert its own suppressive effects against the intimal thickening of the blood vessel at said PTCA or PTA-treated site thereof.

Therefore, there is described a photodynamic therapy method of suppressing such thickening of the vascular

intima in the blood vessel wall and also such vascular restenosis of the blood vessel which are inducible after the angioplasty treatment of the blood vessel has been done, which method comprises:

- administering mono-L-aspartylchlorin e6 of the formula (I)

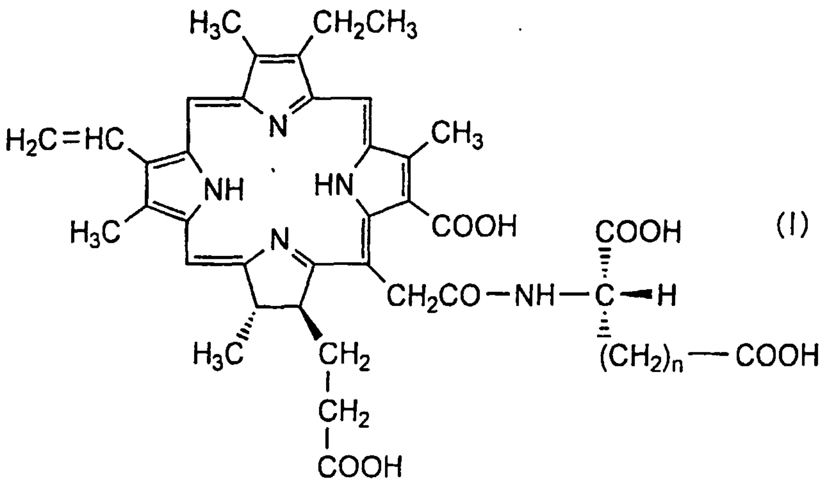

(I)

wherein n is 1, or a salt thereof, to a patient whose blood vessel has received the treatment by angioplasty;
making the administration of the compound of the formula (I) at a dosage so adjusted that a therapeutically effective amount of the compound of formula (I) can accumulate in the cell layers of the blood vessel wall at the site of the blood vessel having received the treatment by angioplasty;
inserting percutaneously and transluminally into and locating in the interior of said blood vessel at the position of the site thereof having received the treatment by angioplasty, such a laser-irradiating device that comprises a balloon catheter having a central and longitudinal hole therein and having an inflatable balloon made of a laser-transmissive material at the front end of said catheter and that comprises a laser-irradiating optical fiber so arranged as to extend within and through said central and longitudinal hole in the balloon catheter and is equipped on the catheter with an inlet tube for introduction of an inflating liquid to be sent into the interior space of said inflatable balloon; and adjusting the position of the balloon catheter within the blood vessel so that said balloon of the balloon catheter is located oppositely to the angioplasty-treated site of the blood vessel;
making said balloon of the balloon catheter inflate by delivery of the inflating liquid in the interior space of the balloon of the catheter via said inlet tube for introduction of the inflating liquid into the balloon interior space of the catheter of said device, thereby to produce the inflated balloon in the balloon catheter ;
allowing the central axis of the laser-irradiating optical fiber present within the central and longitudinal hole of said balloon catheter to be held coincidently with and in the same position as the central axis of the vascular lumen of the blood vessel at the angioplasty-treated site of the blood vessel, with aid of such a supporting force which is generated by said inflated balloon to be exerted on the balloon catheter and on the inner wall of the blood vessel at said angioplasty-treated site;
and irradiating the compound of formula (I) having accumulated in the interior of the blood vessel wall positioned at the angioplasty-treated site of the blood vessel, with a laser light of an appropriate wavelength, by transmitting from a laser-generator the laser light via said optical fiber in the balloon catheter, in such a manner that the transmitted laser light is emitted outwardly from the laser-emitting part at the front end of said optical fiber and is made to pass through the liquid medium present in the inflated balloon and through the wall material of said inflated balloon of the balloon catheter, so that the emitted laser light irradiates the compound of formula (I) present in the blood vessel inner wall, whereby said compound so irradiated is photoactivated and allowed to generate and exert the suppressive effects thereof against the thickening of the vascular intima in the angioplasty-treated site of the blood vessel.

In order to assemble or construct the laser-irradiating device which comprises the balloon catheter and is used in the method described in this invention and in which the laser-irradiating and transmitting optical fiber is provided and arranged in the interior of the hole of the known type of balloon catheter, it is possible to employ anyone of the commercially available or known optical fibers which are usually used for the laser irradiation in the PDT treatment of tumors formed in the esophageal mucous membrane or pulmonary trachea. The optical fiber as employed may be inserted and arranged in an appropriately way in the central longitudinal hole present in a commercially available or known balloon catheter. The balloon catheter has usually been employed in conventional methods for conducting a percutaneous transluminal

coronary angioplasty (PTCA) or percutaneous transluminal angioplasty (PTA).

In the method described in this invention, commercially available or known devices usable for PDT may be used appropriately, in combination, to assemble or construct from them the laser-irradiating device which is to be used in the present method. Any of the laser-irradiating devices may be constructed and used in the method of the invention, as long as the laser-irradiating device so constructed is able to achieve its performances that can satisfy the following three criteria: 1) the balloon membrane of the balloon catheter as arranged in said device is made of a laser-transmissive material; 2) the central axis of the internal optical fiber for transmission of the irradiating light can be held coincidently with and in the same position as that of the central axis of the vascular lumen of the blood vessel, by making the balloon inflated, so that the irradiating laser having emitted from said optical fiber is allowed to be applied evenly onto the blood vessel inner wall; and 3) the flowing of the bloodstream can be stopped in the blood vessel by the inflated balloon.

When a blood vessel having a large caliber, such as the aorta of lower limbs, is to be treated as a target for PDT, there may be employed also such balloon catheter having the laser-irradiating optical fiber and having the balloon, which is commercially available to be used in the PDT treatment of pulmonary cancer and esophageal carcinomas.

In the method described in this invention, it is possible to effect the laser irradiation by means of a laser-irradiating device in which a linear optical fiber for irradiation of laser has been inserted in the central hole of a commercially available balloon catheter known for use in the various intervention procedures. For example, in "Eur J Vasc Endovasc Surg" Vol.16(4), p.284-291 (1988), there is disclosed a method for effecting the laser irradiation by means of such a laser-irradiating device which has been assembled by removing a guide-wire out of the central hole of the balloon catheter conventionally used for PTA and then inserting a linear optical fiber in the empty hole of the balloon catheter in place of said guide-wire.

Similarly, when the coronary artery is treated as a target for PTCA, it is possible that there is employed such a balloon catheter which is equipped with a guidewire and has the same structure as that of a commercially available balloon catheter usable conventionally for PTCA, and of which the balloon is made of a laser transmissive-material, and also it is possible that the PTCA procedures are then effected by means of said balloon catheter, and that subsequently to the PTCA procedures, a laser-irradiating linear optical fiber of a thin diameter is inserted through the guidewire-introducing inlet and slot of said balloon catheter into the central hole of the balloon catheter so as to assemble a laser-irradiating device which may be employed for carrying out the method described in this invention.

In addition, it is possible that an X-ray marker made of a suitable X-ray-shielding material is attached to one end of the laser-irradiating optical fiber for the purpose of permitting the position of the optical fiber to be detectable by X-ray radiography. Additionally, use may be made of such a laser-irradiating device which has a structure so modified as to permit the blood stream of the coronary artery to be maintained at a suitable level, if said device can meet the purposes as intended in the method described in the invention. Currently, when a balloon catheter is used in angioplasty, concomitant use of a stenting device can provide some success. The PDT method described in this invention may be also carried out in association with a stenting device which has been implanted in the blood vessel.

Figure 1 of the appended drawings shows a diagrammatical view of the longitudinal cross-section of such a laser-irradiating device which comprises a balloon catheter and can be used in the method described in this invention and which is insertable into the interior of the blood vessel. The laser-irradiating device of Figure 1 comprises the balloon catheter (1) and an optical fiber (3) for transmission and irradiation of the laser, wherein the front end of the optical fiber (3) is equipped with a laser emitter (3’) which connects with the optical fiber (3). The optical fiber (3) with its laser emitter (3’) has been inserted and located firmly in the interior of the central and longitudinal hole of said balloon catheter (1) which has a sheath structure and is made of synthetic resin, which is commonly used for the treatment according to percutaneous transluminal coronary angioplasty (PTCA) or percutaneous transluminal angioplasty (PTA), which is provided with a cylindrical and empty jacket (1’) and with the central and longitudinal hole (6) (not shown). Further, the optical fiber (3) has been adhered to the inner wall (1") of the hole of the balloon catheter (1), so that the laser-irradiating device so assembled has a wholly integrated structure.

In the structure of the device of Figure 1, the laser emitter (3’) connected to the optical fiber (3) is so arranged that the emitter (3’) is placed in a position to oppose to the balloon-forming part (2) of the balloon catheter (1). Towards the rear end of the balloon catheter (1) is provided an inlet tube (4) for introduction of the inflating liquid (for example, sterile physiological saline) which is to be injected and delivered into the empty jacket (1’) of the balloon catheter in order to inflate the inflatable balloon of the catheter. When the inflating liquid has been injected into the jacket (1’) and delivered in the balloon-forming part (2), the balloon-forming part (2) can be extended and inflated by the hydraulic pressure of the inflating liquid to produce the expanded balloon of the balloon catheter.

Figure 2 of the appended drawings shows a diagrammatical view of the longitudinal cross-section of the laser-irradiating device of Figure 1 and the cross-section of the blood vessel, when the said device have had the expanded or inflated balloon (2’) which has been produced by inflation of the balloon-forming part (2) with the inflating liquid as injected and delivered after the previous insertion of the laser-irradiating device of Figure 1 in the blood vessel (5).

As shown in Figure 2, the balloon catheter (1), which is equipped with the optical fiber (3) and provided in the laser-irradiating device, has the completely inflated balloon (2’), of which the top surface has been brought into a close

contact with the inner wall (5') of the blood vessel (5) with being pressed outwardly under the resulting hydraulic pressure of the inflating liquid as delivered to fill the inflated balloon (2'). The completely inflated balloon (2') has generated and exerted the force for supporting the optical fiber (3) of the balloon catheter, since it has received the hydraulic pressure of the inflating liquid as delivered therein. Thus, the laser-irradiating device as a whole is allowed to be located firmly in the interior of the blood vessel in such way that the central axis of the optical fiber (3) can be held and maintained coincidently with and in the same position as that of the central axis of the vascular lumen of the blood vessel.

Figure 3 of the appended drawings shows a diagrammatical view of the longitudinal cross-section of a sheath-insertable balloon catheter made of synthetic resin, which is commonly used for PTCA or PTA and which is provided with a cylindrical and empty jacket (1') and also with a central and longitudinal hole (6) therein. The balloon catheter as shown in Figure 3 has such structure that it may be formed by depriving the optical fiber and its laser emitter from the laser-irradiating device as shown in Figure 1.

The balloon catheter (1) shown in Figure 3 has an empty jacket (1'), a balloon-forming part (2), an inlet tube (4) for introduction of the inflating liquid and a central and longitudinal hollow hole (6). The balloon catheter shown in Figure 3 may be firstly inserted percutaneouly and transluminally into the blood vessel by means of a usual sheath usable for intravascular insertion of the balloon catheter. By subsequently inserting a guide-wire in the hollow hole of the balloon catheter and using said guide-wire as a guide according to the known technique of PTCA or PTA, said balloon catheter once inserted as above may then be moved to reach such site of the blood vessel which is predetermined to be treated by the method described in this invention. Subsequently, said balloon catheter as inserted is located firmly in position at said site to be treated. Thereafter, the guide-wire is drawn out off from the hole of said balloon catheter. An optical fiber of a thin diameter is then inserted in and through the emptied hollow hole (6) of the so located balloon catheter to fill the inner hole of the balloon catheter, so that a laser-irradiating device usable in the method described in this invention can be assembled and constructed by combining the necessary members of said laser-irradiating device within the blood vessel.

Next, the procedures for using the laser-irradiating device of Figure 1 and for carrying out the method described in this invention will be described.

Thus, the compound of the formula (I), mono-L-aspartylchlorin e6 or its salt, is administered at its appropriate dose to a patient whose blood vessel has received a therapeutic treatment by PTCA or PTA. Estimation is then made of a time point at which an effective amount of the administered compound has accumulated in the PTCA or PTA-treated site of the blood vessel. With choosing an optimal timing nearly before or after said time point which was estimated as above, the laser-irradiating device of Figure 1 is then introduced at the optimal timing into the interior of said blood vessel percutaneously and transluminally by the PTCA or PTA technique.

The positions of the balloon-forming part of the balloon catheter and the laser emitter of the optical fiber in the laser-irradiating device as introduced are then adjusted so that the balloon-forming part and the laser emitter are located in a position opposite to the angioplasty-treated site of the blood vessel which has been predetermined to be treated by the method described in this invention. Said site is namely the blood vessel site having previously been treated by PTCA or PTA. Then, the body of the laser-irradiating device is held firmly within the blood vessel. Next, the inflating liquid (for example, sterile physiological saline or physiological saline containing an X-ray contrast medium) is delivered and introduced into the balloon-forming part of the balloon catheter through the aforesaid inlet tube of the device under an appropriate liquid pressure. Thereby, the balloon-forming part of the balloon catheter is made to inflate and to produce the inflated balloon. The outer wall of the completely inflated balloon as filled with said liquid can thus be brought into a close contact with the inner wall of the blood vessel and has occupied the inner room of the vascular lumen of the blood vessel, to intercept the flowing of the bloodstream in the vascular lumen.

Then, the laser light is made to pass into the optical fiber of said device and is allowed to be transmitted therethrough and emitted from the emitter of the optical fiber and to pass across the inflating liquid medium present in the inflated balloon and also across the wall membrane of the inflated balloon, so that the inner wall of the blood vessel at the PTCA or PTA-treated site thereof can be irradiated with the irradiating laser light.

The administered chlorin e6 compound which has accumulated in the cells of the blood vessel inner wall at said PTCA or PTA-treated site can then be photo-activated by the irradiating laser light as emitted, to bring about an apoptosis of the vascular smooth muscle cells and to exert the suppressive effects against the intimal thickening which would be inducible in the PTCA or PTA-treated site of the blood vessel.

After making the irradiation of laser light for a given period of time, the inflating liquid is discharged from the inflated balloon and from the device used. Subsequently, the whole of the laser-irradiating device is withdrawn out from the blood vessel. Then, the routine post-treatments are carried out to finish the therapeutic treatment as intended.

## BRIEF DESCRIPTION OF THE DRAWINGS

Figure 1 of the appended drawings shows a diagrammatical view of the longitudinal cross-section of the laser-irradiating device which comprises a balloon catheter and can be used in the method described in this invention.
Figure 2 shows a diagrammatical view of the longitudinal cross-section of the laser-irradiating device which comprises a balloon catheter having its balloon inflated after the insertion of the device of Figure 1 in the blood vessel.
Figure 3 shows diagrammatical view of the longitudinal cross-section of a balloon catheter which is insertable intravascularly and may be used to assemble and construct within the blood vessel such a laser-irradiating device that is available in the method of described in this invention.
Figure 4 shows a diagrammatical view of the traverse cross-section of the blood vessel at a site thereof having the intimal thickening as produced in the lower abdominal artery of rabbit which was tested in Example 2 described hereinafter.

BEST MODE FOR CARRYING OUT THE INVENTION

The method described in this invention will now be illustrated with reference to the following Examples.

Example 1

Mono-L-aspartylchlorin e6 tetrasodium salt (NPe6) was intravenously administered at a dose of 2.5 mg/kg to a rabbit weighing about 4 kg. A commercially available 2F balloon catheter was immediately then inserted through the femoral artery into the abdominal aorta of rabbit. Thereafter, there are repeated operations of drawing out from the blood vessel the balloon catheter with the inflated balloon, inserting the balloon catheter again into the blood vessel, and drawing out the balloon catheter with the inflated balloon, whereby a rabbit model with an arterial injury was produced. This method for producing the rabbit model having the arterial injury at the blood vessel inner wall was in accordance with the method of Hsiang et al. (see Ann. Vasc. Surg., Vol.9, p.80-86, 1995).

At the end of 3 hours after the administration of NPe6, an optical fiber for irradiation of laser (made of an acrylic polymer filament having an external diameter of 0.5 mm and provided with the emitter part of 15mm in length) was inserted into the hollow hole of the 2F balloon catheter which had been inserted in and kept remained at the balloon-injured site of the abdominal aorta blood vessel. The balloon of the catheter was then inflated. Laser light of 664 nm wavelength was passed into and transmitted through the optical fiber to make the laser light to irradiate the abdominal artery at the balloon-injured site, and thereby to carry out the PDT treatment of said site at a laser fluence of 50 or 100 $J/cm^2$. The laser (of the wavelength of 664 nm) used was emitted from a laser-generating source of semi-conductor type known for PDT.

At the respective ends of four days, two weeks and four weeks after the above PDT treatment so made, the site having received the PDT treatment was pathologically studied. According to the pathological findings as obtained at the end of four days after the PDT treatment comprising the above-mentioned intravascular laser irradiation method, it was observed that the cells of the tunica media of the blood vessel at the PDT-treated site thereof were destroyed in a broad region of ranging from the inner wall side towards the outer wall side of the blood vessel. According to the pathological findings as obtained at the respective ends of 2 and 4 weeks after the PDT treatment, it was observed that the intimal thickening as produced by the vascular neo-intima formed at the PDT-treated site was inhibited, and that no injuries were developed in the regions of the blood vessel which are participating in the entire construction of the blood vessel.

Example 2

Mono-L-aspartylchlorin e6 tetrasodium salt was intravenously administered at a dose of 2.5 mg/kg to three rabbits each weighing about 4 kg. Thereafter, there are repeated the operations of inserting the 2F balloon catheter through the femoral artery into the abdominal aorta vessel of rabbit, inflating the balloon of the inserted catheter, and drawing out the catheter from the abdominal aorta vessel with keeping the balloon inflated, whereby the rabbit model with the arterial injury was produced according to the aforesaid method of Hsiang et al.

Figure 4 of the appended drawings shows the diagrammatical view of the traverse cross-section of the blood vessel positioned at the site thereof having the intimal thickening which was produced as above in the lower abdominal artery of the rabbit and which was tested as the rabbit model of the arterial injury in this study. As shown in Figure 4, the blood vessel (5), which is seen at the position of the blood vessel site having the intimal thickening, has comprised the vascular adventitia (7), the tunica media (8) and the thickening intima, namely the neo-intima (9) as well as the vascular lumen (10). The neo-intima (9) had newly been produced from the inner side of the tunica media (8) due to the injury as induced by the balloon-intervention.

At the end of three hours after the administration of mono-L-aspartylchlorin e6 tetrasodium salt, a device comprising an optical fiber for the irradiation of laser was inserted in the blood vessel of rabbit having the intimal thickening,

to arrive at the balloon-injured site of the blood vessel. In order to estimate in comparison the achievable suppressive effects of the PDT treatments against the vascular restenosis, the above test procedures were conducted with employing two different types of the laser-irradiating devices comprising the optical fiber for the irradiation of laser, a first one type of which two different devices was such a laser-irradiating device that comprised an optical fiber as integrated with a balloon catheter and that is shown in Figure 1 of the appended drawing. The other type of the laser-irradiating device employed was comprising simply the conventional type of cylindrical optical fiber. In the tests, either one of these two different types of the laser-irradiating devices containing an optical fiber was inserted into the abdominal aorta under test. Under roentgenoscopy, an X-ray-shielding marker which had been provided in the laser-irradiating optical fiber so inserted was observed as a guide. This guide was used so that the position of the optical fiber device as inserted could be adjusted so as to be located in a position optimal to make the laser to irradiate selectively just the balloon-injured site of the abdominal aorta under test. Then, the irradiation of the laser through said optical fiber was carried out at a laser fluence of 50 or 100 J/cm$^2$ by means of a semiconductor generator for generating the laser of a wavelength of 664 nm. However, when the first type of the laser-irradiating device comprising the optical fiber integrated with the balloon catheter shown in Figure 1 was employed for the intravascular irradiation of laser, there was interposed, prior to the laser-irradiation step, such additional steps that a sterile physiological saline was injected into the inlet tube of the inserted balloon catheter via a pressure pump and was delivered into the interior space of the balloon of the balloon catheter to make the balloon inflated. There was involved a completed inflation of the balloon of the balloon catheter comprised in said device as suitably located in the blood vessel. The completely inflated balloon then could produce such conditions that the flowing of the bloodstream was intercepted at said balloon-injured site of the blood vessel. When these conditions of the interception of the intravascular flowing of the bloodstream had been obtained by means of said completely inflated balloon, the irradiation of laser was then commenced to be effected. These procedures of carrying out the PDT treatment with employment of the laser-irradiating device of Figure 1 are greatly different from the procedures as conducted in the case when the second type of the laser-irradiating device comprising simply the conventional type of cylindrical optical fiber was employed for the intravascular irradiation of laser. At the end of two weeks after the above operations of PDT, the PDT-treated site of the blood vessel was pathologically studied. From the photographies of showing the traverse cross-section of histopathologically stained test specimens as collected from the above PDT-treated site of the blood vessel, the area of the tunica media and the area of the neo-intima of the test specimens of the blood vessel under test were measured by an image analyzer. The ratio of the area of the tunica media to the area of the neo-intima of the blood vessel specimens was calculated. Using the so calculated ratio as an index, the inhibition rate (%) of the intimal thickening was estimated. In the control tests (untreated), the third rabbit under test has received neither administration of NPe6, nor irradiation of the laser.

The inhibition rate (%) above was calculated from the following equation:-

$$\text{Inhibition rate (\%)} = \frac{A - B}{A} \times 100$$

wherein A means the ratio of the area of neo-intima to the area of tunica media in the control group (Untreated group) of rabbit under test; and B means the ratio of the area of neo-intima to the area of tunica media in the treated group of rabbits under test.

The test results so obtained are tabulated in Table 1 below.

Table 1

| Suppressive effects of PDT (photodynamic therapy) treatment according to the method of this invention on the intimal thickening induced at the balloon-intervented and injured site of blood vessel | | |
|---|---|---|
| Features of Treatment | Ratio of area of neointima to area of tunica media in blood vessel | Inhibition rate (%) |
| Drug administered and Manner of irradiation of laser | | |
| Untreated Group (with no administration of drug and with no PDT treatment) | 1.22±0.32 | - |

(continued)

| Suppressive effects of PDT (photodynamic therapy) treatment according to the method of this invention on the intimal thickening induced at the balloon-intervented and injured site of blood vessel | | |
|---|---|---|
| Features of Treatment | Ratio of area of neointima to area of tunica media in blood vessel | Inhibition rate (%) |
| Drug administered and Manner of irradiation of laser | | |
| Treated Group (comparative) with administration of 2.5 mg/kg of NPe6 and with laser-irradiation by the conventional type of cylindrical optical fiber device for irradiation of laser at influence of 100 J/cm$^2$ | 0.87±0.37 | 28.7 |
| Treated Group (inventive) with administration of 2.5 mg/kg of NPe6 and with laser-irradiation by the optical fiber device integrated with the balloon catheter for irradiation of laser at influence of 50 J/cm$^2$ | 0.68±0.21 | 44.3 |

As clearly seen from the results of Table 1, it is confirmed that the PDT method, which was conducted with employing the laser-irradiating device comprising the optical fiber and the balloon catheter in an integral combination as shown in Figure 1, that is, the laser-irradiating device of Figure 1 comprising the optical fiber as integrated with the balloon catheter and capable of intercepting the intravascular flowing of the bloodstream, is able to achieve the suppressive effects on the vascular restenosis at the angioplasty-treated site of the blood vessel, to an enhanced extent than the PDT method which was conducted with employing a laser-irradiating device comprising simply the conventional type of cylindrical optical fiber.

## INDUSTRIAL APPLICABILITY

As described above, this invention provides a use of the compound of the formula (I) or its salt as an active ingredient for the preparation of a vascular restenosis-suppressing composition in combination with a laser-irradiating device which comprises a balloon catheter and a laser-irradiating optical fiber as integrated with each other. This composition is useful for use in a photodynamic therapy to suppress the vascular restenosis inducible in the blood vessel at a site thereof having been treated by PTA or PTCA. In addition, as compared with the therapeutic effects obtainable by the conventional methods, more uniform and better therapeutic effects of PDT can be obtained according to the method described in this invention.

## Claims

**1.** Use of mono-L-aspartyl chlorin e6 or a pharmaceutically acceptable salt thereof, as a suppressant or preventive agent for the preparation of a composition to be used in combination with a laser irradiating device comprising a balloon catheter to be positioned firmly on an inner wall of a blood vessel at an angioplasty dilated site, in photodynamic therapy for treating a vascular restenosis which is inducible at said angioplasty-dilated site of said blood vessel as dilated by a percutaneous transluminal coronary angioplasty or by a percutaneous transluminal angioplasty,

- said balloon catheter having a central and longitudinal hole therein and having an inflatable balloon made of a laser-transmissive material at the front end of said catheter and being equipped on the catheter with an inlet tube for introduction of an inflating liquid to be sent into the interior space of said inflatable balloon,
- said laser irradiating device comprising a laser-irradiating optical fiber so arranged as to extend within and through a central and longitudinal hole in the balloon catheter,
- the central axis of the laser-irradiating optical fiber present within the central and longitudinal hole of said balloon catheter being allowed to be held coincidently with and in the same position as the central axis of the vascular lumen of the blood vessel at the angioplasty-treated site of the blood vessel, and
- wherein in the photodynamic therapy treatment an intravascular irradiation is made with a laser light of 560

nm to 760 nm at a laser fluence of 1 to 50 J/cm$^2$, and with keeping said balloon completely inflated to allow said laser-irradiating optical fiber being held coincidently with and in the same position as the central axis of said vascular lumen.

2. Use according to claim 1, wherein the treatment of the inducible vascular restenosis comprises intravascular laser irradiation to said angioplasty-dilated site of said blood vessel in the walt of which said administrated chlorin e6 compound has accumulated, with said intravascular laser irradiation being effected through a wall material of said inflated balloon of said balloon catheter as intravascularly inserted into said blood vessel to such position that said inflated balloon locates to be opposite to and firmly on said vascular inner wall of said blood vessel at said dilated site thereof, whereby an inducible thickening of the vascular intima in said dilated site is inhibited.

## Patentansprüche

1. Verwendung von Mono-L-aspartylchlorin e6 oder eines pharmazeutisch annehmbaren Salzes davon als Hemmstoff oder präventives Mittel zur Herstellung einer Zusammensetzung, die in Kombination mit einer Laserbestrahlungsvorrichtung, die einen Ballonkatheter für seine feste Anordnung an einer Innenwand eines Blutgefäßes an einer durch Angioplastie aufgeweiteten Stelle aufweist, bei der photodynamischen Therapie zur Behandlung einer vaskulären Restenose zu verwenden ist, die an der durch Angioplastie aufgeweiteten Stelle des genannten Blutgefäßes induzierbar ist, das durch perkutane transluminale Coronarangioplastie oder durch eine perkutane transluminale Angioplastie aufgeweitet ist,

- wobei der genannte Ballonkatheter ein darin zentral und in Längsrichtung verlaufendes Loch und einen aufblasbaren Ballon, der aus einem laserdurchlässigen Material hergestellt ist, am Vorderende des genannten Katheters aufweist und an dem Katheter mit einem Einlaßrohr zur Einführung einer Aufblasflüssigkeit versehen ist, die in den Innenraum des genannten aufblasbaren Ballons geleitet werden soll,
- wobei die genannte Laserbestrahlungsvorrichtung eine optische Faser für die Laserbestrahlung umfaßt, die so angeordnet ist, dass sie sich in einem zentralen und in Längsrichtung verlaufenden Loch in dem Ballonkatheter und durch dieses hindurch erstreckt,
- wobei die zentrale Achse der optischen Faser für die Laserbestrahlung, die in dem zentralen und in Längsrichtung verlaufenden Loch des genannten Ballonkatheters angeordnet ist, in einer mit der Zentralachse des vaskulären Lumens des Blutgefäßes an der durch Angioplastie behandelten Stelle des Blutgefäßes zusammenfallenden und gleichen Position gehalten werden kann, und
- wobei bei der photodynamischen Therapiebehandlung eine intravaskuläre Bestrahlung mit einem Laserlicht von 560 nm bis 760 nm bei einer Laserfluenz von 1 bis 50 J/cm$^2$ erfolgt, und wobei man den genannten Ballon in einem vollständig aufgeblasenen Zustand hält, um die optische Faser für die Laserbestrahlung mit der Zentralachse des genannten vaskulären Lumens zusammenfallend und in der gleichen Position zu halten.

2. Verwendung nach Anspruch 1, wobei die Behandlung der induzierbaren vaskulären Restenose eine intravaskuläre Laserbestrahlung der durch Angioplastie aufgeweiteten Stelle des Blutgefäßes umfaßt, in dessen Wand sich die genannte verabreichte Chlorin eb-Verbindung akkumuliert hat, wobei die genannte intravaskuläre Laserbestrahlung durch ein Wandmaterial des genannten aufgeblasenen Ballons des genannten Ballonkatheters erfolgt, während dieser intravaskulär bis zu einer solchen Position in das Blutgefäß eingeschoben ist, dass sich der genannte aufgeblasene Ballon gegenüber und fest auf der genannten vaskulären Innenwand des genannten Blutgefäßes an dessen aufgeweiteter Stelle befindet, wodurch eine induzierbare Verdickung der vaskulären Intima an der aufgeweiteten Stelle inhibiert wird.

## Revendications

1. Utilisation de mono-L-aspartyl chlorine e6 ou d'un sel pharmaceutiquement acceptable de celle-ci en tant qu'agent de suppression ou de prévention pour la préparation d'une composition à utiliser en combinaison avec un dispositif de rayonnement laser comprenant un ballon cathéter à positionner fermement sur une paroi interne d'un vaisseau sanguin au niveau d'un site dilaté d'angioplastie, dans une thérapie photodynamique pour traiter une resténose vasculaire pouvant être induite au niveau dudit site dilaté d'angioplastie dudit vaisseau sanguin dilaté par une angioplastie transluminale percutanée coronarienne ou par une angioplastie transluminale percutanée,

- ledit ballon cathéter comportant un trou central et longitudinal et ayant un ballon gonflable constitué d'un

matériau transmissif du laser au niveau de l'extrémité avant dudit cathéter et équipé sur le cathéter d'un tube d'entrée pour l'introduction d'un liquide de gonflage devant être envoyé dans l'espace intérieur dudit ballon gonflable,
- ledit dispositif de rayonnement laser comprenant une fibre optique de rayonnement laser agencée de sorte à s'étendre dans et à travers un trou central et longitudinal dans le ballon cathéter,
- l'axe central de la fibre optique de rayonnement laser présente dans le trou central et longitudinal dudit ballon cathéter pouvant être maintenu en coïncidence avec et dans la même position que l'axe central de la lumière vasculaire du vaisseau sanguin au niveau du site traité d'angioplastie du vaisseau sanguin, et
- dans laquelle, dans le traitement de thérapie photodynamique, un rayonnement intravasculaire est effectué avec une lumière laser de 560 nm à 760 nm à un flux intégré du laser de 1 à 50 $J/cm^2$ et en gardant ledit ballon complètement gonflé pour permettre à la fibre optique de rayonnement laser d'être maintenue en coïncidence avec et dans la même position que l'axe central de ladite lumière vasculaire.

**2.** Utilisation selon la revendication 1, dans laquelle le traitement de la resténose vasculaire inductible comprend un rayonnement laser intravasculaire audit site dilaté d'angioplastie dudit vaisseau sanguin dans la paroi duquel ledit composé chlorine e6 s'est accumulé, ledit rayonnement laser intravasculaire étant effectué à travers un matériau de paroi dudit ballon gonflé dudit ballon cathéter inséré par voie intravasculaire dans ledit vaisseau sanguin à une position telle que ledit ballon gonflé se situe à l'opposé de et fermement sur ladite paroi interne vasculaire dudit vaisseau sanguin au niveau de son site dilaté, moyennant quoi un épaississement inductible de l'intima vasculaire dans ledit site dilaté est empêché.

FIG. 1

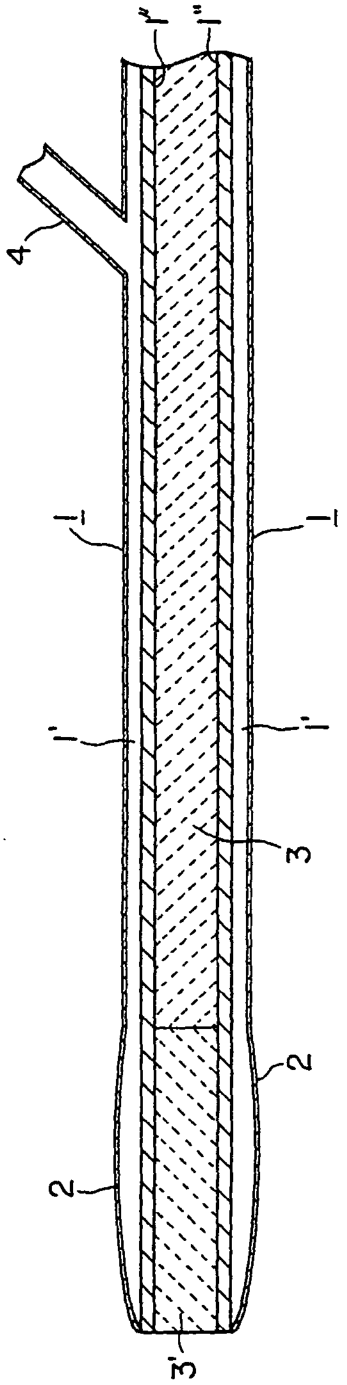

FIG. 2

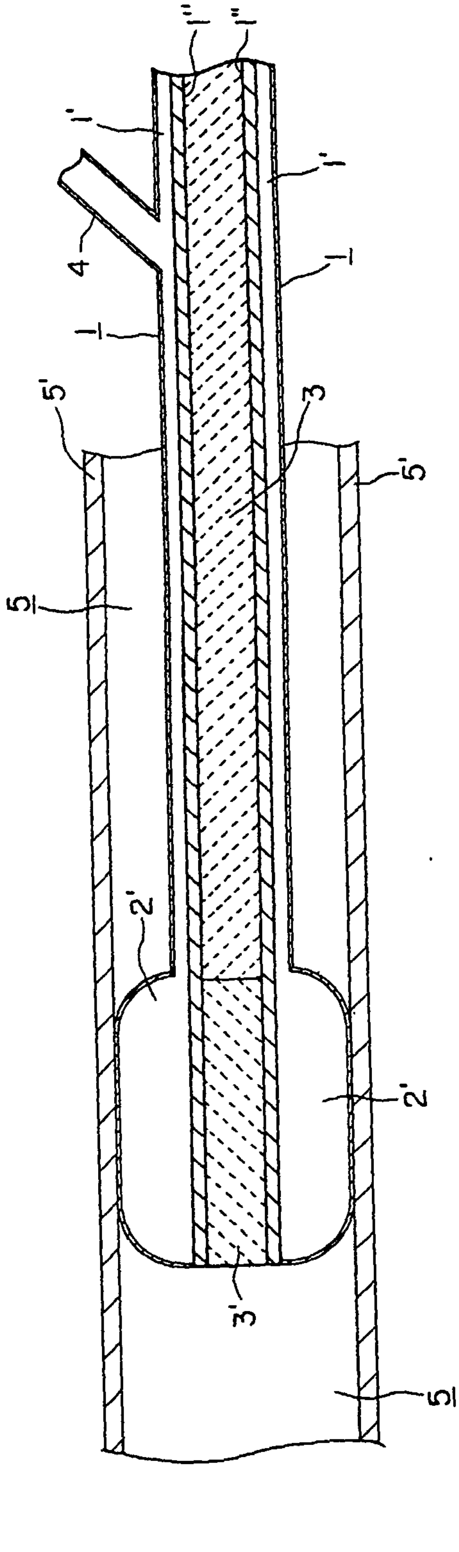

FIG. 3

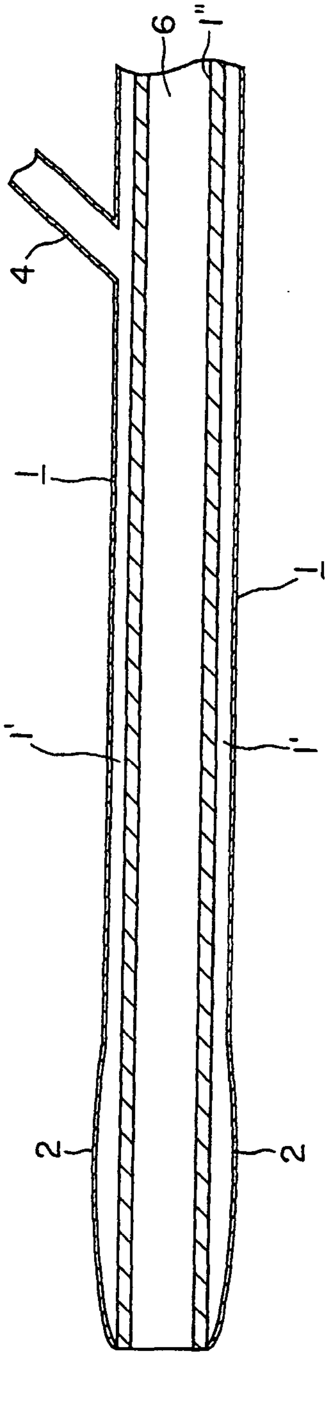

FIG. 4

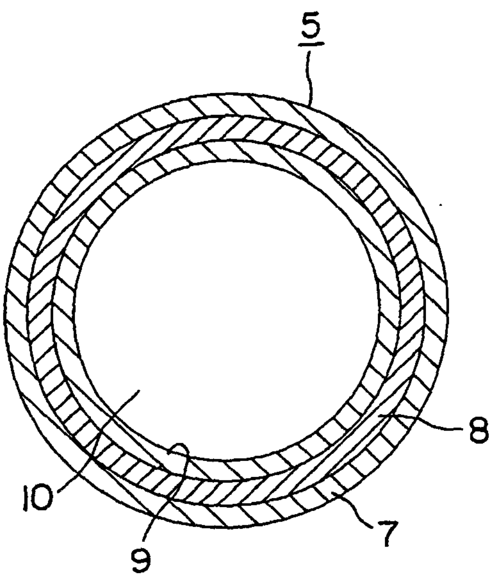

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*


### Patent documents cited in the description

- JP 6088902 A
- JP 6089000 A
- JP 4330013 A
- US 5308861 A
- JP 9188619 A
- JP 8176013 A
- JP 7025768 A

### Non-patent literature cited in the description

- *Ann. Vasc. Surg.,* 1999, vol. 9, 80-86
- *Cardicovasc. Surg.,* 1995, vol. 3, 489-494
- *Japanese journal: Kekkan to Naihi,* 1996, vol. 6, 56-64
- *Dtsch. Med. Wschr,* 1998, vol. 123, 840-846
- *THE JOURNAL OF JAPAN SOCIETY FOR LASER SURGERY AND MEDICINE,* 1997, vol. 18, 295-300
- *The 62nd Scientific Meeting of Japanese Circulation Society,* 1988, 465
- *Eur J Vasc Endovase Surg,* 1988, vol. 16 (4), 284-291
- **HSIANG et al.** *Ann. Vasc. Surg.,* 1995, vol. 9, 80-86